# EUROPÄISCHE PATENTSCHRIFT

(11) **EP 2 873 393 B1**
(45) Veröffentlichungstag und Bekanntmachung des Hinweises auf die Patenterteilung: **03.05.2017**
(21) Anmeldenummer: 14192790.5
(22) Anmeldetag: 12.11.2014
(51) Int. Cl.: A61F 2/50, A61F 2/80, A61F 2/76

(54) **Verfahren zur Bestimmung der Bemaßung eines Prothesenschaftes für einen Gliedmaßen-Stumpf und Verfahren zur Herstellung einer Prothese für einen Gliedmaßen-Stumpf**
Method for determining the dimensioning of a prosthesis shaft for a limb stump and method for producing a prosthesis for a limb stump
Procédé de détermination du dimensionnement d'une emboîture de prothèse pour la réception d'un moignon et procédé de fabrication d'une prothèse pour la réception d'un moignon

(30) Priorität: 19.11.2013 DE 102013223572
(43) Veröffentlichungstag der Anmeldung: 20.05.2015
(73) Patentinhaber: Pohlig GmbH, 83278 Traunstein (DE)
(72) Erfinder: Pohlig, Kurt, 83278 Traunstein (DE); Schäfer, Michael, 83278 Traunstein (DE)
(74) Vertreter: Pfenning, Meinig & Partner mbB

(56) Entgegenhaltungen:
- EP-A1- 1 843 291
- DE-A1-102005 008 605
- DE-A1-102008 032 992
- JP-A- 2006 255 199
- US-A- 5 539 649
- GOH J C H ET AL: "Development of an integrated CAD-FEA process for below-knee prosthetic sockets", CLINICAL BIOMECHANICS, BUTTERWORTH SCIENTIFIC LTD, GUILDFORD, GB, Bd. 20, Nr. 6, 2005, Seiten 623-629, XP027795119, ISSN: 0268-0033 [gefunden am 2005-07-01]

## Beschreibung

Die vorliegende Erfindung betrifft ein Verfahren zur Bestimmung der Bemaßung eines Prothesenschaftes für einen Gliedmaßen-Stumpf. Sie betrifft weiterhin ein Verfahren zur Herstellung einer Prothese für einen Gliedmaßen-Stumpf unter Verwendung des erwähnten Verfahrens.

Bei der Herstellung einer Prothese für einen Gliedmaßen-Stumpf ist es wesentlich, die Prothese optimal an den Gliedmaßen-Stumpf anzupassen. Hierzu wird, wie im Folgenden anhand eines Beispiels eines Oberschenkelstumpfes beschrieben wird, herkömmlicherweise zuerst der Oberschenkelumfang auf verschiedenen Höhen (d.h. an Umfangs-Linien in etwa oder vollständig senkrecht zur Längserstreckung des Oberschenkelknochens) vermessen. Dies kann beispielsweise im Abstand von 3 cm parallel zueinander erfolgen. Zusätzlich wird auf diesen Höhen eine Bestimmung des Maßes der Eindellung des Gewebes ermittelt, die auftritt, wenn das Maßband einer definierten Zugkraft unterworfen wird. Alternativ dazu kann auch die Zugkraft gemessen werden, die zur Erreichung eines bestimmten Maßes an Einschnürung erforderlich ist. Der Orthopädie-Techniker ermittelt aus dem Oberschenkelumfang und der Zugkraft/Einschnürungsmessung das sogenannte Reduktionsmaß (RM). Dieses Reduktionsmaß wird dann vom Orthopädie-Techniker zur Bemaßung und Konstruktion des Schaftes der Prothese verwendet, so dass der Prothesenschaft optimal an den vermessenen Oberschenkelstumpf und dessen Eigenschaften angepasst ist.

Problematisch bei diesem Stand der Technik ist, dass die Bestimmung des Reduktionsmaßes eine subjektive Komponente bei der Messung des Oberschenkelumfangs und/oder bei der Zugkraftmessung enthält. Diese ist letztlich abhängig von der Person, die das Reduktionsmaß misst und damit nicht vollständig reproduzierbar. Das herkömmliche, händische Verfahren zur Bemaßung eines Prothesenschaftes ermöglicht es also nicht, vergleichbare oder gar einheitliche hohe Standards bei der Konstruktion des Schaftes zu gewährleisten.

Als weitere Möglichkeit des Standes der Technik zur Bemaßung des Prothesenschaftes wird von dem Gliedmaßen-Stumpf ein Gipsabdruck abgenommen, der dann anschließend bei der Schaftformung verwendet wird. Auch dieses Verfahren ist stark von der ausführenden Person abhängig und nicht gesichert produzierbar.

Als weiteres Verfahren im Stand der Technik wird die Computertomografie oder Magnetresonanztomografie eingesetzt. Mit diesen lassen sich dreidimensionale Daten über Größe, Form und Lage von Knochen und Weichteilen ermitteln. Aus diesen Daten kann ebenfalls die Bemaßung des Schaftes ermittelt werden. Nachteilig an diesem Verfahren sind der hohe gerätetechnische Aufwand und die hohen Kosten, die für die tomografische Bildgebung erforderlich sind. Weiterhin hat dieses Verfahren den Nachteil, dass bei diesen tomografischen bildgebenden Verfahren der Gliedmaßen-Stumpf nur im entspannten Zustand, d.h. ohne Muskelkontraktion, erfasst werden kann.

Die JP 2006 255199 A offenbart ein 3D-Form-Messinstrument für eine Vorrichtung zur Herstellung einer Prothese, welches ein Mittel zum Aussenden von Ultraschallwellen, ein Mittel zum Detektieren von reflektierten Wellen der Ultraschallwellen, ein Mittel zum Bestimmen der Position des Mittels zum Aussenden der Ultraschallwellen und des Mittels zum Detektieren der reflektierten Wellen der Ultraschallwellen, ein Mittel zum Analysieren von detektierten Daten, um eine Grenze zwischen verschiedenen Geweben eines menschlichen Körpers zu erhalten, und ein Mittel zum Erstellen eines FE-Modells von einem analysierten Ergebnis aufweist. Die Vorrichtung zur Herstellung einer Prothese wird zur Entwicklung einer Prothese unter Verwendung dieser Daten verwendet.

Die EP 1 843 291 A1 offenbart ein Verfahren zur Herstellung eines Prothesenschafts. Das Verfahren umfasst Separieren der Anteile von Knochen-, Muskel- und Fettgewebe aus einer dreidimensionalen Darstellung des Stumpfs, Bestimmen einer Ziel-Kompression des Stumpfs auf der Grundlage des Gewichts des Patienten und der Außenfläche des Stumpfs, Bestimmen einer Vielzahl von Querschnitten des Stumpfs, basierend auf der Ziel-Kompression und einer jeweils vorgegebenen Kompressibilität der Anteile von Muskel- und Fettgewebe, und Erstellen eines stetigen dreidimensionalen Modells des Prothesenschafts aus der Vielzahl von Querschnitten. Die DE 10 2008032992 A1 offenbart ein Verfahren und System zur Erstellung eines 3-D-Schaft-Stumpfmodells zur Herstellung eines Prothesenschaftes zur Verbindung eines
einen Stumpf bildenden Körperteils mit einer Prothese, wobei die Schritte umfassen: Akquirieren von dreidimensionalen (3-D) Bilddaten des den Stumpf bildenden Körperteils, das mehrere Gewebearten umfasst; Segmentieren der 3-D-Bilddaten zur Bestimmung der Verteilung von wenigstens einer Gewebeart des Stumpfes; Rekonstruieren eines 3-D-Schaft-Stumpfmodells anhand der segmentierten 3-D-Bilddaten, das Geometrie des Stumpfes und Verteilung der wenigstens einen segmentierten Gewebeart des Stumpfes beschreibt; Modifizieren des 3-D-Schaft-Stumpfmodells anhand von wissensbasierten Regelsätzen zur Anpassung des 3-D-Schaft-Stumpfmodells an den Stumpf, wobei die wissensbasierten Regelsätze die in dem 3-D-Schaft-Stumpfmodell enthaltenen Informationen über Geometrie des Stumpfes und/oder Verteilung der wenigstens einen segmentierten Gewebeart berücksichtigen und eine oder mehrere Regeln umfassen, die eine oder mehrere Eigenschaften der wenigstens einen segmentierten Gewebeart verwenden.

Die DE 10 2005 008 605 A1 offenbart ein Verfahren zur Herstellung einer äußeren Prothese oder Orthese mit den Schritten: Erstellen einer Schichtaufnahme des betreffenden Körperteils, Umwandeln der erstellten Daten in ein 3-D-Modell, Auswerten der Knochen-, Muskel- und Fettgewebestruktur, Ermitteln von Kompressionszonen in Abhängigkeit von der Auswertung und Erstellen eines Prothesen-/Orthesenelements in Abhängigkeit von diesen Daten.

Die US 5 539 649A offenbart ein System zur automatisierten Entwicklung und Herstellung von künstlichen Beinen. Das System besteht aus einer Anordnung von Ultraschallwandlern, die speziell konstruiert sind, um das verbliebene Bein eines Amputierten zu beschallen. Die hierbei gewonnenen Flugzeit-Informationen werden anschließend in einem Computer gespeichert, wo sie verarbeitet werden, um dreidimensionale Informationen hinsichtlich der relativen Lage der Bein-, Haut- und Knochenoberflächen im Raum zu liefern. Diese dreidimensionalen Daten werden dann verwendet, um ein Bild des Beines mit einem computergestützten Entwicklungssystem für eine individuelle prothetische Entwicklung und Herstellung zu erzeugen.

In "Development of an integrated CAD-FEA process for below-knee prosthetic sockets", Goh, J.C.H. et al., Clinical Biomechanics, Butterworth, Bd. 20, Nr. 6, 2005, Seiten 623-629, XP027795119, ISSN: 0268-0033 ist ein Verfahren offenbart, das eine Finite-Elemente-Analyse in CAD integriert. Dies ermöglicht eine objektivere Einschätzung der Passform eines Prothesenschafts und verbessert die Chance einer erfolgreichen ersten Anpassung. Aufgabe der vorliegenden Erfindung ist es daher ein Verfahren zur Bestimmung der Bemaßung eines Prothesenschaftes und ein Verfahren zur Herstellung einer Prothese für einen Gliedmaßen-Stumpf zur Verfügung zu stellen, das schnell, kostengünstig und in hohem Grade reproduzierbar durchgeführt werden kann.

Diese Aufgabe wird durch ein Verfahren zur Bestimmung der Bemaßung eines Prothesenschaftes nach Anspruch 1 sowie durch ein Verfahren zur Herstellung einer Prothese nach einem der Ansprüche 8 oder 9 gelöst. Vorteilhafte Weiterbildungen der erfindungsgemäßen Verfahren werden in den jeweiligen abhängigen Ansprüchen gegeben.

Bei dem erfindungsgemäßen Verfahren wird anhand eines Modells die Bemaßung eines Prothesenschaftes für einen Gliedmaßen-Stumpf ermittelt. Alternativ können auch anhand des genannten Modells die maßgeblichen Reduktionsmaße (RM) des Gliedmaßen-Stumpfes bestimmt werden. Sowohl anhand der Bemaßung des Prothesenschaftes oder auch anhand der so ermittelten Reduktionsmaße kann ein Orthopädie-Techniker dann erfindungsgemäß die Prothese für den Gliedmaßen-Stumpf herstellen. Dies kann in herkömmlicher Weise handwerklich erfolgen. Andererseits ist es auch möglich, zuerst anhand dieser Werte ein digitales Modell des Prothesenschaftes zu erstellen und danach dann diesen manuell oder automatisiert zu fertigen. Die Herstellung der Prothese kann mit Verfahren wie Tiefziehen, Gießen oder Rapid Prototyping erfolgen, die im Stand der Technik zur Herstellung von Werkstücken bekannt sind.

Zur Erstellung des Modells des Gliedmaßen-Stumpfes wird erfindungsgemäß zuerst die Oberfläche des Gliedmaßen-Stumpfes gescannt und erfasst. Dies kann beispielsweise durch optische Triangulation erfolgen. Dieser Schritt stellt eine 3D-Darstellung der Oberfläche des Gliedmaßen-Stumpfes zur Verfügung.

In einem weiteren Schritt werden sonographisch an mindestens zwei in Erstreckungsrichtung des Gliedmaßen-Stumpfes voneinander benachbarten Querschnitten durch den Gliedmaßen-Stumpf die Verteilung verschiedener Gewebetypen innerhalb des Gliedmaßen-Stumpfes bestimmt. Hierzu eignen sich sonographische Verfahren wie insbesondere Ultraschallmessungen besonders, da diese eine Unterscheidung der einzelnen Gewebetypen (Muskelgewebe, Bindegewebe, Knochen etc.) ermöglichen. Erfindungsgemäß wird auf diese Weise die Verteilung verschiedener Gewebetypen nicht nur innerhalb lediglich eines einzelnen Querschnitts bestimmt, sondern für mehrere zueinander beabstandete Querschnitte. Beabstandet bedeutet in diesem Falle in Erstreckungsrichtung des Gliedmaßen-Stumpfes, beispielsweise in Längsrichtung des Oberschenkelknochen beabstandet zueinander. Beispielsweise können solche Messungen in regelmäßigen Abständen, beispielsweise 3 cm, beabstandet zueinander erfolgen. Die Bestimmung der Verteilung der verschiedenen Gewebetypen in Querschnitten durch den Gliedmaßen-Stumpf dient insbesondere zur Beurteilung der Kompressibilität des Gliedmaßen-Stumpfes an den entsprechenden Stellen.

Anhand der in dem Scan-Schritt erfassten 3D-Daten über die Oberfläche des Gliedmaßen-Stumpfes und anhand der in dem sonographischen Schritt erfassten Daten über die Verteilung verschiedener Gewebetypen in Querschnitten des Gliedmaßen-Stumpfes, wobei die Querschnitte zueinander längs der Erstreckungsrichtung des Gliedmaßen-Stumpfes beabstandet sind, wird dann in einem Modellierungs-/Modellerstellungsschritt ein Modell des Gliedmaßen-Stumpfes erstellt.

In dem Modellerstellungsschritt/Modellierungsschritt wird dann in dieses Modell ein Modell der Knochen der Gliedmaße eingepasst. Dieses Knochen-Modell ist sowohl in seiner Position und Form veränderbar als auch in seiner Größe skalierbar. Dieses Knochenmodell kann daher derart eingepasst werden, dass es bezüglich seiner Lage und seiner Größe mit den Daten über die Lage des Knochengewebes aus dem sonographischen Schritt in bestmöglicher Übereinstimmung ist. Dies bedeutet, dass für alle Patienten lediglich ein standardisiertes Knochenmodell erforderlich ist, das anhand der gemessenen Werte in das Modell eingepasst und an die anatomischen Verhältnisse des Prothesenträgers angepasst wird. Hierdurch können die Bemaßung des Prothesenschaftes oder die Reduktionsmaße des Gliedmaßen-Stumpfes noch zufriedenstellender bestimmt werden.
Anhand dieses so hergestellten Modells des Gliedmaßen-Stumpfes ist es nun möglich, an jeder beliebigen Stelle des Modells des Gliedmaßen-Stumpfes, insbesondere auch an den Stellen des Gliedmaßen-Stumpfes, an denen ein Orthopädie-Techniker herkömmlicherweise die RM-Werte bestimmt, die RM-Werte zu bestimmen. Alternativ kann auch direkt aus diesem Modell die Bemaßung des Prothesenschaftes ermittelt werden.

Bei dem erfindungsgemäßen Verfahren wird also ein Scan der Oberfläche des Gliedmaßen- Stumpfes und eine sonographische Untersuchung des Gliedmaßen-Stumpfes eingesetzt. Alle diese Schritte und Verfahren sind rasch, ohne großen Geräteaufwand und kostengünstig durchführbar. Das erfindungsgemäße Verfahren liefert dabei RM-Werte bzw. die Bemaßung des Prothesenschaftes, die mindestens gleichwertig mit den aus dem Stand der Technik bekannten Verfahren sind, wenn nicht sogar diesen deutlich überlegen, sind. Insbesondere ist es beim erfindungsgemäßen Verfahren möglich, sowohl den Scan-Schritt als auch ggfs. den sonographischen Schritt bei kontrahiertem Muskel durchzuführen. Alternativ ist es auch möglich, einen oder beide dieser Schritte sowohl bei kontrahiertem Muskel als auch bei relaxiertem Muskel durchzuführen. Dies ermöglicht es, weitere und bessere Informationen über das Reduktionsmaß des Gliedmaßen-Stumpfes an verschiedenen Stellen zu ermitteln.

Erfindungsgemäß ist es möglich, das Scannen des Gliedmaßen-Stumpfes mittels Triangulation, Laserabtastverfahren oder dergleichen durchzuführen. Als sonographisches Verfahren kann eine herkömmliche Ultraschallabtastung erfolgen. Diese kann vorteilhafter Weise ventral, dorsal, lateral und medial, oder auch zusätzlich dazwischen, in einem Querschnitt durch den Gliedmaßen-Stumpf die Verteilung verschiedener Gewebetypen innerhalb des Gliedmaßen-Stumpfes ermitteln. Insbesondere kann der Abstand des Muskelgewebes, des Fettgewebes und/oder des Knochengewebes von der Oberfläche des Gliedmaßen-Stumpfes bestimmt werden. Für den Modellierungsschritt eignet sich zur Modellierung des Gliedmaßen-Stumpfes insbesondere ein Finite-Elemente-Modell (FE-Modell).

Wesentlich Unterschied zum Stand der Technik ist, dass das Reduktionsmaß, anhand dessen ein Orthopädie-Techniker eine Prothese herstellt, nicht mithilfe eines Maßbands und unter Ausübung eines bestimmten Zuges ermittelt wird. Vielmehr wird das Reduktionsmaß bzw. unmittelbar die Bemaßung des Prothesenschaftes anhand von Messergebnissen ermittelt, die auf ScanVerfahren und sonographischen Verfahren beruhen. Diese sind reproduzierbar und unabhängig von der Person, die das Verfahren durchführt. Insgesamt ist dadurch die Bestimmung der Reduktionsmaße bzw. der Bemaßung eines Prothesenschaftes erheblich reproduzierbarer und unabhängiger von der Person des die Messung ausführenden geworden. Insbesondere ermöglicht dies auch, über eine Vielzahl von verschiedenen Mitarbeitern eine reproduzierbare gleichmäßig hohe Qualität der letztlich hergestellten Prothese zu garantieren.

Im Gegensatz zum Stand der Technik wird also das Reduktionsmaß oder die Bemaßung des Prothesenschaftes nicht unmittelbar gemessen sondern aus objektivierten Messdaten bestimmt. Außerdem kann die Modellerstellung weitgehend automatisiert erfolgen. Das Verfahren ist dadurch wesentlich schneller als die herkömmlichen Verfahren im Stand der Technik.

Das in das Modell des Gliedmaßen-Stumpfes, in das die sonographischen Daten und die dreidimensionalen Oberflächen-Scandaten eingearbeitet sind, einzupassende Knochenmodell ist dabei ein Modell der in der jeweiligen Gliedmaße vorliegenden Knochen. Dabei ist jedoch durch dieses Knochenmodell zwar die relative Lage der Knochen untereinander und/oder die äußere Gestalt der Knochen vorgegeben. Allerdings ist das Modell als skalierbares Modell ausgebildet, so dass je nach Größe des Patienten dieses Knochenmodell in der Größe skaliert und in seiner Form verändert werden kann. Weiterhin kann dieses Knochenmodell als solches in dem Gliedmaßen-StumpfModell gedreht und verschoben werden, so dass es optimal in die gemessenen Werte aus dem sonographischen Schritt und dem Scan-Schritt eingepasst werden kann. Damit genügt ein einzelnes Knochenmodell für jede Art von Patienten. Hierdurch wird die erforderliche Rechenleistung zur Einpassung des Knochenmodells in das Gliedmaßen-Stumpfmodell deutlich verringert und die Einpassung des Knochenmodells deutlich beschleunigt.

Optional ist es zusätzlich auch noch möglich, dass mithilfe der so bestimmten Reduktionsmaße oder mithilfe der so ermittelten Bemaßung des Prothesenschaftes der Prothesenschaft zusammen mit dem Gliedmaßen-Stumpf als Modell simuliert werden. Durch eine derartige Simulation ist es dann möglich, bereits vorab den perfekten Sitz des Prothesenschaftes zu ermitteln. Es ist auch möglich, sofern potentielle Druckstellen zwischen Gliedmaßen-Stumpf und Prothesenschaft erkannt werden, das Modell des Prothesenschaftes weiter zu überarbeiten, um den später gefertigten Prothesenschaft noch besser an den Gliedmaßen-Stumpf anzupassen. Auch diese Simulation kann mittels der Finite-Elemente-Methode erfolgen.

Im Folgenden werden einige Beispiele erfindungsgemäßer Verfahren gegeben. Darin bezeichnen gleiche und ähnliche Bezugszeichen gleiche und ähnliche Elemente, so dass ggfs. deren Beschreibung weggelassen wird.

Es zeigen
Figur 1 ein Ablaufschema eines herkömmlichen Verfahrens;
Figur 2 ein Ablaufschema für die Durchführung eines ersten erfindungsgemäßen Verfahrens;
Figur 3 ein Ablaufschema für die Durchführung eines zweiten erfindungsgemäßen Verfahrens; und
Figur 4 zeigt eine konkrete Durchführung des erfindungsgemäßen Verfahrens;
Figur 5 zeigt den Oberflächenscan eines Gliedmaßenstumpfes entsprechend dem in Figur 4 gezeigten Beispiel;
Figur 6 zeigt die Modellierung eines Gliedmaßenstumpfes gemäß dem Verfahren aus Figur 4;
Figur 7 zeigt die mit dem Verfahren gemäß Figur 4 gewonnen Werte.

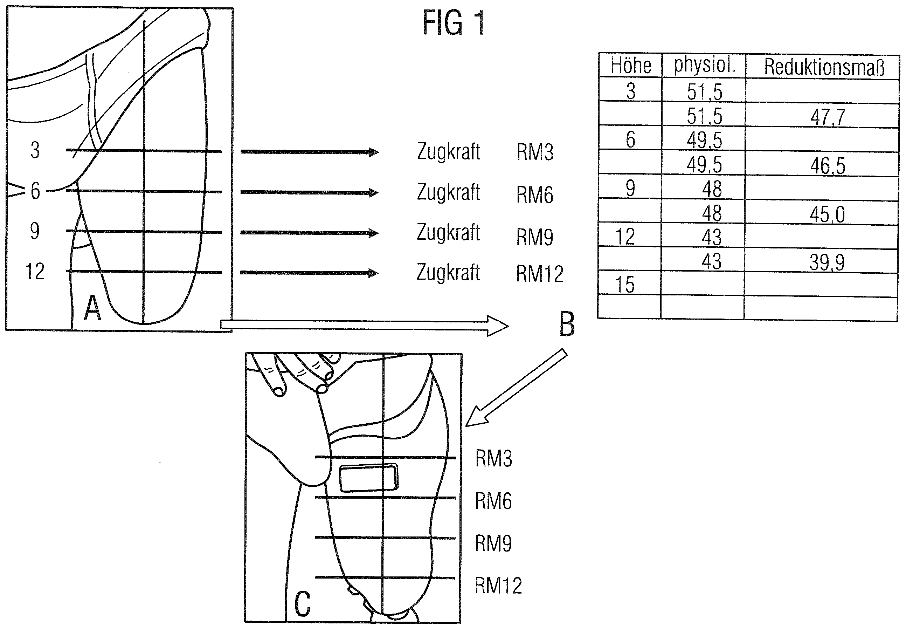

Figur 1 zeigt das Ablaufschema eines herkömmlichen Verfahrens zur Bestimmung von Reduktionsmaßen. Wie in Teilfigur 1a dargestellt, wird hierzu zuerst der Umfang des Oberschenkelstumpfes mittels eines Maßbandes an verschiedenen, gleichmäßig voneinander beabstandeten Umfängen des Oberschenkelstumpfes bestimmt. Dabei wird die Zugkraft konstant gehalten, sodass die an verschiedenen Umfanglinien bestimmten Umfänge miteinander vergleichbar sind. Die Umfangslinien, an denen die Umfänge bestimmt werden, sind dabei jeweils 3 cm voneinander beabstandet und verlaufen senkrecht zur Stumpflängsachse beginnend vom Tuber distal 3 cm in den vordefinierten Abständen von je 3 cm. Figur 1B zeigt die aus dieser manuellen Messung mittels eines Maßbandes bestimmten Werte.

An den entsprechenden Umfangslinien, die mit den Zentimeterangaben 3, 6, 9 und 12 bezeichnet sind, wird hier zuerst das physiologische Umfangsmaß bestimmt, das ohne Zug und ohne jede Reduktion des Umfangs mit einem locker angelegten Maßband bestimmt wird. Die Angaben sind in cm. In der dritten Spalte sind dann die Umfangsmaße bei Ausübung eines definierten Zuges von 4 kg auf das Maßband angegeben. Es ist unmittelbar zu erkennen, dass durch die Ausübung der Zugkraft der jeweilige Umfang verringert wird. Die Reduktion entsteht dabei im wesentlichen dadurch, dass das Muskelgewebe in seinem Volumen durch den Zug auf dem Maßband um ca. 10 % reduziert wird. Demgegenüber ist das Fettgewebe in seinem Volumen nicht reduzierbar, jedoch verschieblich.

Das in Spalte 3 angegebene Reduktionsmaß, das auf diese Weise handwerklich bestimmt wird, wird dann verwendet um die in Figur 1C dargestellte Prothese mit Prothesenschaft herzustellen. Durch die Berücksichtigung des Reduktionsmaßes ergibt sich dabei ein fester, jedoch angenehmer Sitz des Prothesenschaftes auf dem Gliedmaßenstumpf.

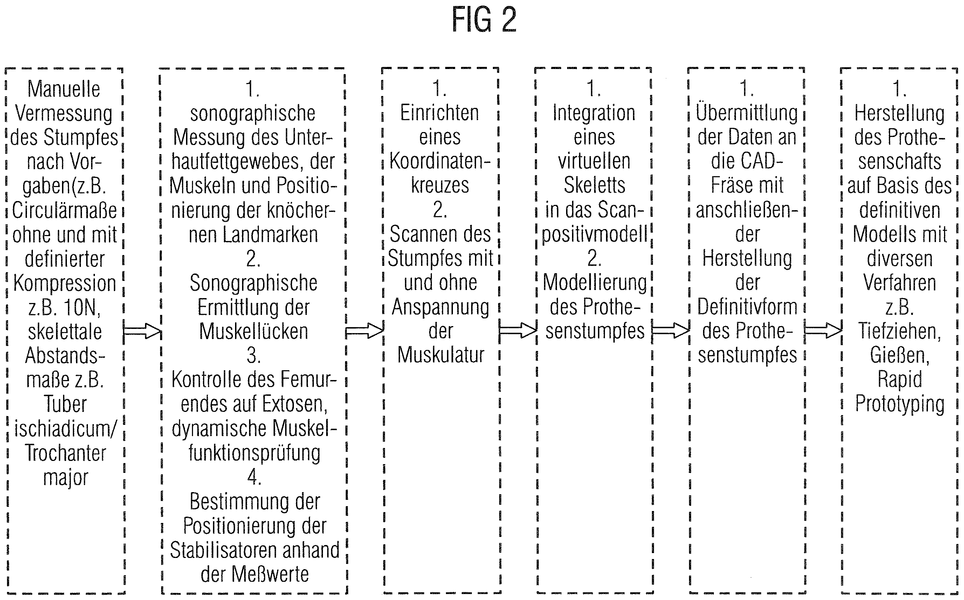

Figur 2 zeigt das erfindungsgemäße Verfahren als Ablaufschema. In einem ersten Schritt wird demnach zuerst eine manuelle Vermessung des Gliedmaßen-Stumpfes durchgeführt. Dabei werden bestimmte Vorgaben eingehalten, z.B. die Bestimmung der Zirkulärmaße ohne und mit definierter Kompression (z.B. 10 N), Bestimmung der skeletalen Abstandsmaße zum Beispiel zwischen Tuber Ischiadicum und Trochanter Major. Dieser Schritt ist optional.

Im nächsten Schritt erfolgt eine sonographische Untersuchung des Gliedmaßen-Stumpfes mithilfe sonographischer Verfahren, insbesondere Ultraschall. Auf die Reihenfolge des Scanschritts und des sonographischen Schritts kommt es dabei nicht immer an. Es ist jedoch günstig, wenn vor dem Scanschritt die Muskellücken und der Verlauf der Muskulatur, mögliche Exostosen usw. festgestellt werden, um diese mit Markern auf dem Scan darzustellen.

Mit der sonographischen Untersuchung wird in verschiedenen, zueinander beabstandeten Querschnitten durch den Gliedmaßen-Stumpf die Verteilung der verschiedenen Gewebetypen ermittelt. Beispielsweise werden Muskellücken, Extosen am Femurende, Positionierung von Fettgewebe oder anderen, knöchernen Landmarken erfasst. Weiterhin kann eine dynamische Muskelfunktionsprüfung mit relaxiertem und kontrahiertem Muskel durchgeführt werden, um die dynamische Verteilung der einzelnen Gewebetypen zu bestimmen.

Anhand dieser Daten ist es auch bereits möglich, (Stumpf-)Stabilisatoren (z.B. Oberflächenprotrusionen) in der späteren Prothese bezüglich ihrer Orte festzulegen. Hierzu eignen sich beispielsweise Muskellücken und dergleichen.

In einem dritten Schritt wird der Gliedmaßen-Stumpf äußerlich dreidimensional gescannt. Auch dieses Scannen und damit Bestimmen der äußeren 3D-Form des Gliedmaßen-Stumpfes kann sowohl mit als auch ohne Anspannung des Muskels erfolgen.

Dazu kann zusätzlich neben dem Gliedmaßen-Stumpf ein Koordinatenkreuz positioniert werden, so dass die Lage einzelner Merkmale des Scans anhand dieses Koordinatenkreuzes präzise erfasst werden kann.

Anhand der sonographischen Messwerte und der 3D-Scandaten wird dann ein Modell des Gliedmaßen-Stumpfes erstellt (Scan-Positiv-Modell). Während oder nach dieser Modellierung kann dann ein Skelettmodell ("virtuelles Skelett") in die Modellierung integriert werden. Das Modell des Skeletts ist dabei sowohl in seiner Position in allen Freiheitsgraden änderbar und zusätzlich in seiner Größe skalierbar. Anhand von knöchernen Landmarken wird z.B. der Hüft-, Becken- und Oberschenkelknochen maßstabgetreu und gegebenenfalls unter Berücksichtigung weiterer bildgebender Verfahren einskaliert.

Nachdem dieses virtuelle Skelett in das Modell des Prothesenstumpfes eingefügt wurde, was insbesondere anhand verschiedener Merkmale der sonographischen Daten erfolgen kann, werden die Daten, nach Abschluss der Modellierung des Stumpfmodells, an eine CAD-Fräse übermittelt. Diese CAD-Fräse erzeugt nun entweder ein Modell des Prothesenstumpfes in der Form, wie dieser sich im adaptierten Prothesenschaft darstellt oder ein Modell des Prothesenschaftes in den dem Prothesenstumpf benachbarten Oberflächenbereichen.

Zur Herstellung des Modells des Prothesenstumpfes oder auch bereits des Prothesenschaftes eignen sich nicht nur Fräsverfahren als Negativverfahren sondern auch Tiefziehverfahren, Gießverfahren oder heutzutage auch Rapid-Prototyping.

In einem alternativen Verfahren werden nicht nur die Bemaßung des Prothesenschaftes bestimmt sondern auch oder alternativ die Reduktionsmaße des Gliedmaßen-Stumpfes. Auch anhand dieser Werte kann dann in herkömmlicher handwerklicher Arbeit oder wie beschrieben durch automatisierte Verfahren wie z.B. Prototyping, Gießen, Tiefziehen oder auch Fräsen ein Prothesenschaft hergestellt werden.

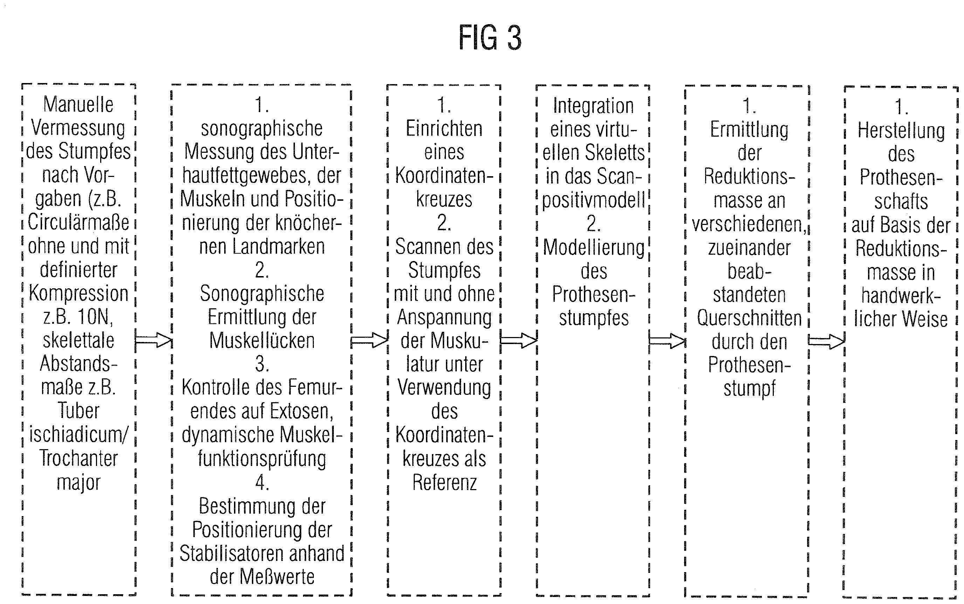

Figur 3 zeigt ein weiteres erfindungsgemäßes Verfahren als Ablaufschema. Dabei sind die ersten vier Schritte identisch mit denjenigen der Figur 2. Dies bedeutet, dass das Modell des Prothesenstumpfes bis einschließlich der Einpassung des virtuellen Skeletts identisch ist. Anhand dieses Modells werden nun jedoch im Unterschied zur Figur 1 die Reduktionsmaße an verschiedenen Stellen des modellierten Prothesenstumpfes bestimmt. Eine derartige Bestimmung kann an denselben Stellen erfolgen, wie sie beim herkömmlichen Bestimmen der Reduktionsmaße längs des Umfangs des Prothesenstumpfes mittels eines Maßbandes erfolgen würde. Dies bedeutet, dass die Reduktionsmaße an verschiedenen, zueinander beabstandeten Querschnitten durch den modellierten Prothesenstumpf anhand der im Modell dargestellten Verteilung verschiedener Gewebe des modellierten Prothesenstumpfes bestimmt werden. Zuletzt wird anhand dieser Reduktionsmaße, beispielsweise in handwerklicher Weise, von einem Orthopädie-Techniker eine Prothese mit Prothesenschaft hergestellt.

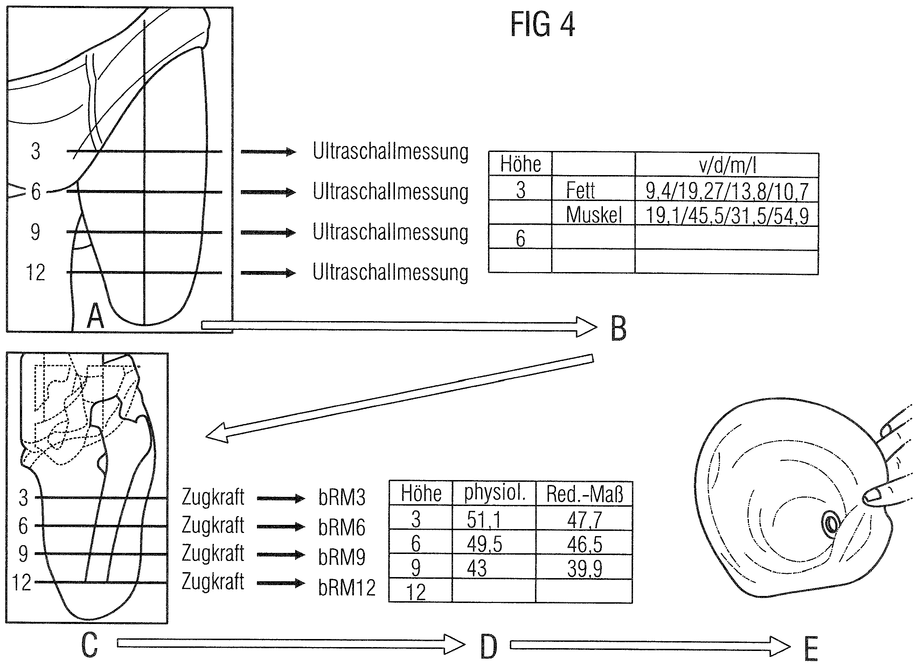

Die Figuren 4 bis 7 zeigen nun ein konkretes Beispiel zur Durchführung des erfindungsgemäßen Verfahrens. Figur 4 zeigt dabei einen Überblick über das Gesamtverfahren.

In Figur 4A sind nun wiederum am Beispiel des in Figur 1 dargestellten Prothesenstumpfes diejenigen Linien eingezeichnet anhand derer im Querschnitt eine Ultraschallmessung des Prothesenstumpfes durchgeführt wird. Die Querschnitte verlaufen hierbei wiederum senkrecht zur Stumpfachse beginnend vom Tuber distal 3cm in in Figur 4A eingezeichneten Abständen von je 3 cm. Mit dieser Ultraschallmessung wurden die Koordinaten der verschiedenen Gewebearten Muskelgewebe/Fettgewebe/Knochengewebe (genauer deren Abstand von der Hautoberfläche) an insgesamt vier verschiedenen Umfangspositionen (ventral, dorsal, medial, lateral (v/d/m/l)) bestimmt.

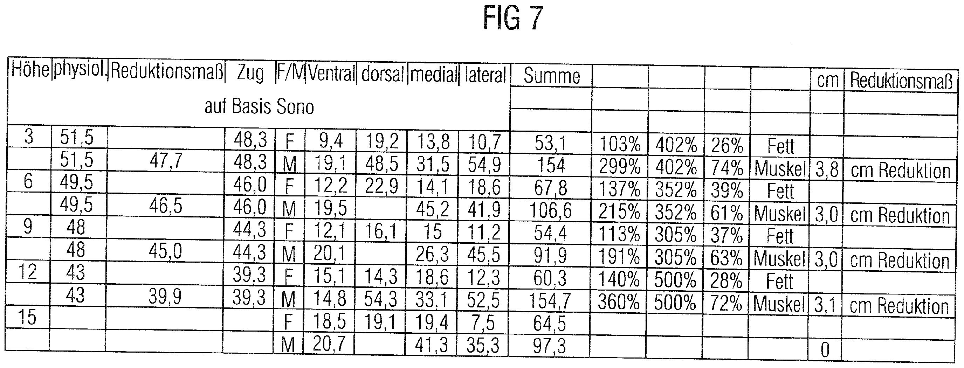

Die entsprechenden Werte werden ausschnittsweise in der Tabelle in Figur 4B gegeben. Die vollständigen Daten finden sich in Figur 7. Demnach hat das Fettgewebe einen Abstand von der Hautoberfläche von 9,4 mm ventral, 19,27 mm dorsal, 13,8 mm medial und 10,7 mm lateral. Das Muskelgewebe weist einen Abstand von der Hautoberfläche im mit 3 bezeichneten Querschnitt von 19,1 mm ventral, 45,5 mm dorsal, 31,5 mm medial und 54,9 mm lateral auf.

Zusätzlich wird mit einem 3D-Scanverfahren die Oberfläche des Gliedmaßenstumpfes erfasst. Anschließend wird aus diesem Oberflächenscan und den mit der Ultraschallmessung ermittelten Werten ein Modell des Gliedmaßenstumpfes erstellt, das in Figur 4C gezeigt ist. Wie in Figur 4C dargestellt wird in dieses Modell dann ein Knochenmodell eingepasst, das skalierbar und in seiner Lage veränderbar ist, sodass es die Lage der Knochen in dem Gliedmaßenstumpf möglichst gut darstellt.

Aus diesem Modell, in dem die Lage der Knochen, der Muskeln und des Fettgewebes bekannt ist, wird nun sowohl der physiologische Umfang (ohne Zug und Kontraktion der Muskeln) und zusätzlich der Umfang unter Anwendung einer simulierten Zugkraft auf ein Maßband als Reduktionsmaß bestimmt. Die ist möglich, da sowohl die Kompressibilität als auch die Verschieblichkeit der einzelnen Gewebearten bekannt ist und so aus dem Modell des Gliedmaßenstumpfes das Reduktionsmaß direkt bestimmbar ist.

Anhand dieses Reduktionsmaßes wird nun automatisiert ein Prothesenschaft hergestellt, der in Figur 4E dargestellt ist. Alternativ können die Reduktionsmaße auch einem Orthopädietechniker zur Verfügung gestellt werden, sodass dieser anhand der Reduktionsmaße in ansonsten herkömmlicher Weise einen Prothesenschaft fertigt.

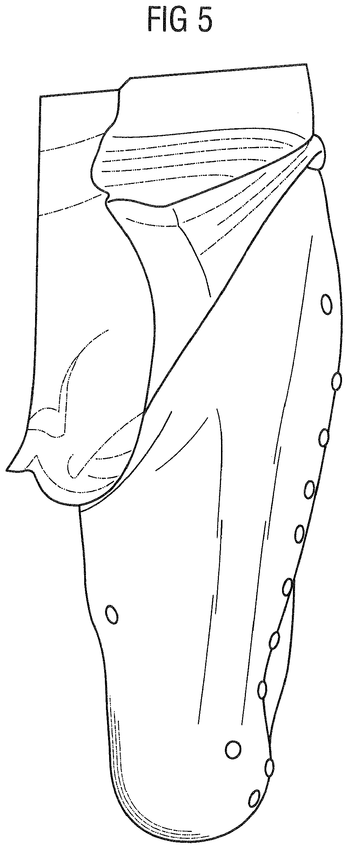

Zur Erläuterung des in Figur 4 dargestellten erfindungsgemäßen Verfahrens ist in Figur 5 der Oberflächenscan des Gliedmaßenstumpfes gezeigt, der in die Modellierung des Gliedmaßenstumpfes in Figur 4C eingeht.

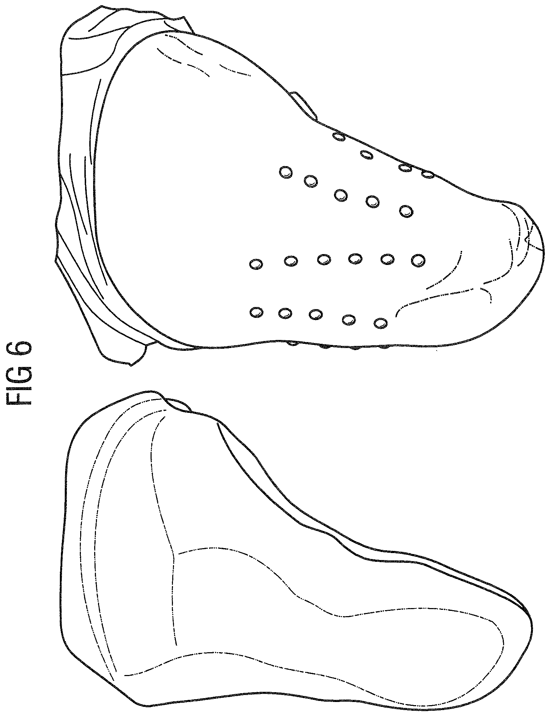

Weiterhin ist in Figur 6 ein Schritt aus der Modellierung des Gliedmaßenstumpfes in Figur 4C dargestellt. Denn dieses Modell wird hier beispielhaft erstellt, indem zuerst anhand der Werte für das Muskelgewebe aus der vorhergehenden Ultraschallmessung ein 3D-Modell des Muskelgewebes erstellt wird. Dies ist in Figur 6 auf der linken Seite zu sehen. Auf dieses Modell des Muskelgewebes wird nun anhand der in der vorhergehenden Ultraschallmessung bestimmten Werte das Fettgewebe aufmodelliert, sodass sich insgesamt die Außenkontur des Gliedmaßenstumpfes modellhaft ergibt.

Figur 7 stellt nochmals zusammenfassend die nummerische Bestimmung des Reduktionsmaßes anhand des erfindungsgemäßen Verfahrens dar.

In der ersten Spalte ist wiederum der Abstand zum Tuber ischiadicum in Zentimetern angegeben, an dem sonographisch die Verteilung des Fett- und Muskelgewebes bestimmt wird.

In der zweiten Spalte ist der physiologische Umfang des Gliedmaßenstumpfes in Doppelmessung in Zentimetern angegeben, wie er aus dem 3D-Scan des Gliedmaßenstumpfes ermittelt wird.

In der vierten Spalte ("zug") sind die Messwerte für das Reduktionsmaß angegeben, wie es mittels eines Maßbandes herkömmlich durch einen Orthopädietechniker bestimmt wird. Diese Werte sind zum Vergleich des erfindungsgemäßen Verfahrens mit dem herkömmlichen Verfahren in der Tabelle angegeben.

In der weiteren Tabelle ist für jeden der bestimmten Umfänge jeweils eine Zeile für Fettgewebe (F) und eine Zeile für Muskelgewebe (M) vorgesehen. Die folgenden vier Spalten geben den Abstand des jeweiligen Gewebes von der Oberfläche der Haut in Millimetern an. Diese Angaben entsprechen denjenigen in Figur 4B. Die Angaben sind dabei aus der sonographischen Messung für insgesamt vier Positionen, nämlich ventral, dorsal, medial und lateral, bestimmt. Die Summe der jeweilig bestimmten vier Werte ist in der nächsten Spalte, die mit "Summe" bezeichnet ist, gegeben. So ist zu erkennen, dass beispielsweise im Abstand 6 cm die Summe für Fettgewebe 67,8 mm beträgt, während sie für Muskelgewebe 106 mm beträgt. Aus diesen Werten kann nun hinreichend genau der Anteil an Fettgewebe und Muskelgewebe bestimmt werden. So ergibt sich beispielsweise im Abstand von 6 cm eine Verteilung aus 39 % Fettgewebe und 61 % Muskelgewebe (entsprechend 67,8 und 106,6 in der vorhergehenden Spalte). Aus dieser Verteilung von Fett- und Muskelgewebe für den jeweiligen Querschnitt durch den Gliedmaßenstumpf kann nun aufgrund der Eigenschaften von Fettgewebe und Muskelgewebe bestimmt werden, wie stark sich der Umfang des Gliedmaßenstumpfes beim Aufbringen einer bestimmten Zugkraft auf ein ggf. anzulegendes Maßband bzw. beim Aufbringen einer bestimmten Druckkraft auf die Haut längs dieses Querschnitts durch den Gliedmaßenstumpf reduziert. Es ergibt sich hier, dass in der Höhe 3 cm der Umfang sich unter dem definierten Druck um 3,8 cm reduzieren wird. Ausgehend von dem physiologischen Umfang von 51,5 cm ergibt sich folglich ein mit dem erfindungsgemäßen Verfahren bestimmtes Reduktionsmaß von 47,7 cm im Querschnitt auf Höhe von 3 cm. Für die weiteren vermessenen Querschnitte in Höhe von 6 cm, 9 cm und 12 cm ergibt sich in gleicher Weise ein Reduktionsmaß von 46,5 cm, 45,0 cm und 39,9 cm.

Vergleicht man das Reduktionsmaß gemäß der vorliegenden Erfindung mit dem herkömmlich bestimmten Reduktionsmaß in der vierten Spalte der Tabelle in Figur 7, so zeigt sich eine Abweichung, die mit der größeren Präzision des erfindungsgemäßen Verfahren zu erklären. Insbesondere sind die in Spalte 4 dargestellten Messwerte vom jeweilig Messenden und anderen äußeren Einflüssen abhängig, sodass sie durchaus auch variieren können. Insgesamt ergibt sich also durch das erfindungsgemäße Verfahren eine von äußeren Einflüssen weitestgehend befreite Bestimmung des Reduktionsmaßes. Anhand dieses Reduktionsmaßes kann dann ein Prothesenschaft erstellt werden, der einen besseren Sitz auf dem Prothesenstumpf aufweist und daher auch einen höheren Tragekomfort besitzt.

## Patentansprüche

1. Verfahren zur Bestimmung der Bemaßung eines Prothesenschaftes für einen Gliedmaßenstumpf,
wobei in einem Scanschritt die Oberfläche des Gliedmaßenstumpfes gescannt und erfasst wird;
in einem sonographischen Schritt für mehrere zueinander beabstandete Querschnitte durch den Gliedmaßenstumpf an mindestens zwei voneinander beabstandeten Stellen längs des Umfangs jedes Querschnitts die Verteilung verschiedener Gewebetypen innerhalb des Gliedmaßenstumpfes bestimmt wird,
in einem Modellerstellungsschritt anhand der in dem Scanschritt und dem sonographischen Schritt erfassten Daten ein Modell des Gliedmaßenstumpfes erstellt wird, in das Modell ein in seiner Position veränderbares und in seiner Größe skalierbares Knochenmodell der in der Gliedmaße des Gliedmaßenstumpfes vorliegenden Knochen eingepasst wird, und in einem Bestimmungsschritt mittels des so erstellten Modells entweder die Bemaßung des Prothesenschaftes unmittelbar bestimmt wird oder die für die Erstellung des Prothesenschaftes maßgeblichen Reduktionsmaße des Gliedmaßenstumpfes bestimmt werden.

2. Verfahren nach dem vorhergehenden Anspruch, **dadurch gekennzeichnet, dass** zur Einpassung des Knochenmodells weitere Daten über die Lage der Knochen in der Gliedmaße erfasst werden.

3. Verfahren nach einem der vorhergehenden Ansprüche, **dadurch gekennzeichnet, dass** der Scanschritt bei kontrahierter Muskulatur und gegebenenfalls zusätzlich bei nicht kontrahierter Muskulatur des Gliedmaßenstumpfes durchgeführt wird.

4. Verfahren nach einem der vorhergehenden Ansprüche, **dadurch gekennzeichnet, dass** das Scannen des Gliedmaßenstumpfes mittels Triangulation, Laserabtastverfahren oder dergleichen erfolgt.

5. Verfahren nach einem der vorhergehenden Ansprüche, **dadurch gekennzeichnet, dass** die Querschnitte einen einheitlichen und konstanten Abstand, vorteilhafterweise 3 cm, zueinander aufweisen.

6. Verfahren nach einem der vorhergehenden Ansprüche, **dadurch gekennzeichnet, dass** in dem sonographischen Schritt für jeden Querschnitt durch den Gliedmaßenstumpf ventral, dorsal, lateral und medial die Verteilung verschiedener Gewebetypen innerhalb des Gliedmaßenstumpfes, insbesondere der Abstand des Muskelgewebes, des Fettgewebes und/oder des Knochengewebes von der Oberfläche des Gliedmaßenstumpfes bestimmt wird

7. Verfahren nach einem der vorhergehenden Ansprüche, **dadurch gekennzeichnet, dass** das in dem Modellerstellungsschritt erstellte Modell des Gliedmaßenstumpfes als Finite-Elemente-Model (FE-Model) erstellt wird.

8. Verfahren zur Herstellung einer Prothese für einen Gliedmaßenstumpf, **dadurch gekennzeichnet,**
**dass** an verschiedenen Stellen des Gliedmaßenstumpfes die Reduktionsmaße des Gliedmaßenstumpfes gemäß einem Verfahren nach einem der vorhergehenden Ansprüche bestimmt werden und anhand der im Scanschritt erfassten Außenkontur der Gliedmaße und der bestimmten Reduktionsmaße entweder a) die Prothese in herkömmlicher Weise gefertigt wird oder b) ein digitales Model der Prothese erstellt und danach die Prothese auf der Basis des digitalen Models der Prothese manuell oder automatisiert gefertigt wird.

9. Verfahren zur Herstellung einer Prothese für einen Gliedmaßenstumpf, **dadurch gekennzeichnet,**
**dass** die Bemaßung des Prothesenschaftes gemäß einem Verfahren nach einem der vorhergehenden Ansprüche bestimmt wird und anhand der bestimmten Bemaßung entweder a) die Prothese in herkömmlicher Weise gefertigt wird oder b) ein digitales Model der Prothese erstellt und danach die Prothese auf der Basis des digitalen Models der Prothese manuell oder automatisiert gefertigt wird.

## Claims

1. Method for determining the dimensioning of a prosthetic socket for a limb stump,
wherein, in a scanning step, the surface of the limb stump is scanned and detected;
in a sonographic step, for a plurality of spaced-apart cross-sections through the limb stump at at least two spaced-apart locations along the circumference of each cross-section, the distribution of different types of tissue within the limb stump is determined,
in a model-creating step, on the basis of the data acquired in the scanning step and the sonographic step, a model of the limb stump is created, a bone model, changeable in its position and scalable in its size, of the bones present in the limb of the limb stump is fitted to the model, and
in,a determination step, by means of the model thus created, either the dimensioning of the prosthetic socket is directly determined or the degrees of reduction of the limb stump required for the creation of the prosthetic socket are determined.

2. Method according to the preceding claim, characterised an that for fitting the bone model further data on the position of the bones in the limb is acquired.

3. Method according to one of the preceding claims, **characterised in that** the scanning step is carried out with contracted musculature and optionally additionally with non-contracted musculature of the limb stump.

4. Method according to one of the preceding claims, **characterised in that** the scanning of the limb stump is done by means of triangulation, laser scanning methods or the like.

5. Method according to one of the preceding claims, **characterised in that** the cross-sections have a uniform and constant spacing, advantageously 3 cm, from one another.

6. Method according to one of the preceding claims, **characterised in that** in the sonographic step, for each cross-section through the limb stump ventrally, dorsally, laterally and medically, the distribution of different types of tissue within the limb stump, in particular the distance of the muscle tissue, the adipose tissue and/or the bone tissue from the surface of the limb stump is determined.

7. Method according to one of the preceding claims, **characterised in that** the model of the limb stump created in the model-creating step is created as a finite element model (FE model).

8. Method for producing a prosthesis for a limb stump, **characterised**
**in that** at different locations of the limb stump the degrees of reduction of the limb stump are determined in accordance with a method according to one of the preceding claims and on the basis of the outer contour of the limb detected in the scanning step and the degrees of reduction determined, either a) the prosthesis is fabricated in a conventional manner or b) a digital model of the prosthesis is created and thereafter the prosthesis is fabricated on the basis of the digital model of the prosthesis manually or in an automated manner.

9. Method for producing a prosthesis for a limb stump, **characterised**
**in that** the dimensioning of the prosthetic socket is determined in accordance with a method according to one of the preceding claims and on the basis of the dimensioning determined, either a) the prosthesis is fabricated in a conventional manner or b) a digital model of the prosthesis is created and thereafter the prosthesis is fabricated on the basis of the digital model of the prosthesis manually or in an automated manner.

## Revendications

1. Procédé de détermination des dimensions d'une tige de prothèse pour un moignon de membre,
moyennant quoi, lors d'une étape de scannage, la surface du moignon de membre est scannée et mesurée ;
lors d'une étape échographique pour plusieurs sections distantes entre elles,'la répartition des différents types de tissus à l'intérieur du moignon est déterminé à travers le moignon au niveau d'au moins deux endroits distants entre eux le long de la circonférence de chaque section,
lors d'une étape de création d'un modèle, à l'aide des données relevées lors de l'étape de scannage et de l'étape échographique, un modèle du moignon est créé et, dans ce modèle, est intégré un modèle osseux, de position variable et échelonnable en taille, des os présents dans le moignon du membre, et
lors d'une étape de détermination, au moyen du modèle ainsi créé, soit les dimensions de la tige de prothèse sont déterminées directement, soit les dimensions de réduction du moignon, déterminantes pour la création de la tige de prothèse, sont déterminées.

2. Procédé selon la revendication précédente, **caractérisé en ce que**, pour l'intégration du modèle osseux, des données supplémentaires concernant la position des os dans le membre sont relevées.

3. Procédé selon l'une des revendications précédentes, **caractérisé en ce que** l'étape de scannage est effectuée lorsque la musculature est contractée et, le cas échéant, également lorsque la musculature du moignon n'est pas contractée.

4. Procédé selon l'une des revendications précédentes, **caractérisé en ce que** le scannage du moignon a lieu par triangulation, procédé de balayage au laser ou autre.

5. Procédé selon l'une des revendications précédentes, **caractérisé en ce que** les sections présentent entre elles une distance unitaire et constante, de manière avantageuse égale à 3 cm.

6. Procédé selon l'une des revendications précédentes, **caractérisé en ce que**, lors de l'étape échographique, pour chaque section, la répartition des différents types de tissus à l'intérieur du moignon, plus particulièrement la distance entre le tissu musculaire, le tissu graisseux et/ou le tissu osseux et la surface du moignon, est déterminée de manière ventrale, dorsale, latérale et médiale à travers le moignon.

7. Procédé selon l'une des revendications précédentes, **caractérisé en ce que** le modèle de moignon créé lors de l'étape de création du modèle est créé sous la forme d'un modèle à éléments finis (modèle FE).

8. Procédé de fabrication d'une prothèse pour un moignon, **caractérisé en ce que**
à différents endroits du moignon, les dimensions de réductions du moignon sont déterminées d'après un procédé selon l'une des revendications précédentes et à l'aide du contour externe, relevé lors de l'étape de scannage des membres et des dimensions de réduction, soit a) la prothèse est fabriquée de manière traditionnelle soit b) un modèle numérique de la prothèse est créé puis la prothèse est fabriquée manuellement ou de manière automatisée sur la base du modèle numérique.

9. Procédé de fabrication d'une prothèse pour un moignon, **caractérisé en ce que**
les dimensions de la tige de prothèse sont déterminées d'après un procédé selon l'une des revendications précédentes et à l'aide des dimensions déterminées, soit a) la prothèse est fabriquée de manière traditionnelle, soit b) un modèle numérique de la prothèse est créé puis la prothèse est fabriquée manuellement ou de manière automatisée sur la base du modèle numérique de la prothèse.
